# EUROPÄISCHE PATENTSCHRIFT

(11) **EP 2 062 540 B1**
(45) Veröffentlichungstag und Bekanntmachung des Hinweises auf die Patenterteilung: **03.04.2013**
(21) Anmeldenummer: 09000066.2
(22) Anmeldetag: 18.04.2007
(51) Int. Cl.: A61B 17/00

(54) **Okkluder**
Occluder
Occluseur

(30) Priorität: 27.04.2006 DE 102006020250; 10.05.2006 DE 102006022000; 03.08.2006 DE 102006036649
(43) Veröffentlichungstag der Anmeldung: 27.05.2009
(62) Teilanmeldung aus: 07724325.1
(73) Patentinhaber: Vueklar Cardiovascular Ltd., Glentirranmuir Kippen Stirling FK8 3HU (GB)
(72) Erfinder: Melzer, Andreas, Prof., 65227 Niedernhausen (DE); Michitsch, Stefan, 45139 Essen (DE)
(74) Vertreter: Von Rohr

(56) Entgegenhaltungen:
- EP-A1- 1 046 375
- WO-A1-99/12478
- US-A1- 2002 099 437
- US-A1- 2006 212 047

## Beschreibung

Die Erfindung betrifft einen Okkluder zum Verschluß von Körperöffnungen des menschlichen oder tierischen Körpers, insbesondere Okkluder zum perkutanen transkatheter Verschluß von Vorhof-Septum-Defekten des menschlichen oder tierischen Herzens nach dem Oberbegriff des Anspruchs 1.

Aus der EP 1 046 375 A1 ist ein Okkluder zum Verschluss von Körperöffnungen des menschlichen oder tierischen Körpers mit wenigstens zwei im Verschlusszustand der Körperöffnung auf gegenüberliegenden Seiten der Körperöffnung angeordneten zumindest bereichsweise gegenüberliegenden Verschlusskörpem und mit wenigstens einem die Verschlusskörper miteinander verbindenden Zwischenstück bekannt. Das Zwischenstück ist im Verschlusszustand zumindest bereichsweise durch die Körperöffnungen durchgeführt, wobei eine einseitige Verbindung zwischen dem Zwischenstück und jedem Verschlusskörper vorgesehen ist. Das Zwischenstück ist im Verschlusszustand mit jedem Verschlusskörper exzentrisch im Randbereich des Verschlusskörpers verbunden, wobei die im Verschlusszustand auf gegenüberliegenden Seiten der Körperöffnung angeordneten gegenüberliegenden Verschlusskörper auf entgegengesetzten Seiten mit dem Zwischenstück verbunden sind.

Aus der WO 99/12478 A1 ist ebenfalls ein Okkluder bekannt, wobei hier gegenüberliegende Stege vorgesehen sind, um ein Zwischenstück des Okkluders mit zwei äußeren Verschlusskörpern des Okkluders zu verbinden.

Das Foramen ovale ist eine Öffnung der Scheidewand (Septum) zwischen linkem und rechtem Herzvorhof beim Fetus und Neugeborenen. Im Regelfall schließt sich der Spalt in den ersten Lebensmonaten. Bei etwa 20% der Menschen allerdings erfolgt der Verschluß unvollständig, es bleibt eine kleine Öffnung zurück. Dieses persistierende Foramen ovale hat keine Auswirkungen auf das Befinden und wird von seinem Träger deshalb nicht wahrgenommen. Unter Umständen begünstigt es jedoch den Eintritt eines Schlaganfalls. Weil das offene Foramen den Übertritt von Blut aus dem rechten in den linken Vorhof ermöglicht, kann der Fall eintreten, daß ein etwa aus einer Beinvenenthrombose angeschwemmtes Blutgerinnsel über den linken Vorhof direkt in den arteriellen Kreislauf und so womöglich ins Gehirn gelangt. Regulär würde es aus dem rechten Herzkompartiment zunächst in den Lungenkreislauf geschickt und in der Lunge ausgefiltert werden.

Zum Verschluß von Septum- oder Gefäßdefekten werden Okkluder eingesetzt, die mit einem Katheter positioniert sowie aktiviert werden können. Ein Okkluder der zuvor beschriebenen Art ist beispielsweise aus der EP-B 1-0 959 777 bekannt. Okkluder dienen z.B. zum Verschluß eines persistierenden Foramen ovale (PFO) oder eines Atriumseptumdefektes, beispielsweise vom Sekundumtyp (ASD II). Die Implantation läuft wie folgt ab. Der Operateur schiebt einen Katheter von der Leiste ausgehend über die untere Hohlvene in den rechten Herzvorhof und von dort durch das offene Foramen ovale in den linken Vorhof vor. Aus dem Stand der Technik sind Okkluder bekannt, die wie ein doppelter Regenschirm zusammengefaltet und von dem Katheter transportierbar sind. Am Zielort im linken Vorhof wird ein schirmartiger Verschlußkörper des Okkluders geöffnet. Dann wird der Katheter in den rechten Vorhof zurückgezogen und ein zweiter schirmartiger Vorschlußkörper des Okkluders geöffnet. Im Ergebnis liegt der Okkluder an beiden Seiten des Vorhofseptums an. Nach Überwachsen mit Herzinnenhaut ist das Foramen ovale dauerhaft verschlossen.

Bei dem zuvor beschriebenen Implantationsverfahren findet die Intervention unter Röntgensicht sowie parallel dazu erfolgender transösophagealer Echokardiographie (TEE) statt: Ein in der Speiseröhre platzierter Schallkopf generiert Bilder des benachbarten Herzens. Zugleich liegen dem Operateur vor dem Eingriff angefertigte kernspintomographische (MR-)Bilder vor. Die MR-Überwachung der Implantation eines Okkluders ist nur sehr eingeschränkt oder gar nicht möglich. Durch den Okkluder kommt es zum Auftreten von Bild-Artefakten, wobei es sich insbesondere um Suszeptibilitätsartefakte und sogenannte RF-Artefakte (radiofrequency artifacts) handelt. Suszeptibilitätsartefakte sind darauf zurückzuführen, daß der Okkluder eine größere Suszeptibilität als das menschliche Gewebe aufweist. RF-Artefakte werden durch die RF-Anregungsimpulse erzeugt. Dabei werden elektrische Ströme durch die zeitveränderliche Magnetfeld-Komponente der RF-Impulse in den Okkluder induziert. Speziell bei Okkluder-Werkstoffen wie Nitinol oder Tantal gewinnen diese Artefakte an Bedeutung. Im Ergebnis kann auf ein Röntgenverfahren zur Bilddarstellung bei der Implantation des Okkluders nicht verzichtet werden, mit den bekannten Nachteilen derartiger Verfahren.

Der aus der EP-B1-0 959 777 bekannte Okkluder weist im übrigen den Nachteil auf, daß im Verschlußzustand die schirmförmigen Verschlußkörper im Randbereich von den Körperwänden abstehen. An diesen Stellen können sich vermehrt Blutgerinnsel bilden, die eine Gefahr für die Gesundheit des Patienten darstellen können. Da die Verschlußkörper bei dem bekannten Okkluder nicht vollflächig an den Körperwänden anliegen, wird das Überwachsen der Verschlußkörper mit Haut erschwert, so daß der vollständige Verschluß der Körperöffnung unter Umständen nicht gewährleistet ist.

Aufgabe der vorliegenden Erfindung ist es daher, einen Okkluder der eingangs genannten Art zur Verfügung zu stellen, der einen sicheren und vollständigen Verschluß einer Körperöffnung gewährleistet.

Die vorgenannte Aufgabe wird durch einen Okkluder mit den Merkmalen von Patentanspruch 1 gelöst.

Erfindungsgemäß ist eine einseitige Verbindung zwischen dem Zwischenstück und jedem Verschlußkörper vorgesehen, wobei das Zwischenstück im Verschlußzustand mit jedem Verschlußkörper exzentrisch im Randbereich des Verschlußkörpers verbunden ist und wobei die im Verschlußzustand auf gegenüberliegenden Seiten der Körperöffnung angeordneten gegenüberliegenden Verschlußkörper auf entgegengesetzten Seiten mit dem Zwischenstück verbunden sind. Der erfindungsgemäße Okkluder weist vorzugsweise zwei Verschlußkörper auf, wobei grundsätzlich auch mehr als zwei Verschlußkörper vorgesehen sein können und ein Verschlußkörper ggf, mehrere Segmente aufweisen kann. Unter dem Begriff "Verschlußkörper" im Sinne der Erfindung werden somit zunächst alle Segmente verstanden, die im Verschlußzustand auf einer Seite der Körperöffnung vorgesehen sind, um eine Abdichtung der Körperöffnung auf dieser Seite zu bewirken. Die Verschlußkörper üben einen Druck auf die defekten Wandteile des Septum- oder Gefäßdefektes aus, was zu einem Zusammenwachsen der Wandteile führt, ohne das es zwingend notwendig ist, einen Gewebeüberzug für den Okkluder vorzusehen. Ein Gewebeüberzug kann nämlich zu Vemarbungen führen und unerwünschte Gewebereaktionen auslösen.

Der Verschlußkörper soll wenigstens einseitig im Randbereich mit dem Zwischenstück verbunden sein. Dadurch wird eine besonders gute Anlage des Verschlußkörpers gegen die die Körperöffnung umgebenden Körperwände nach der Implantation des Okkluders gewährleistet. Die randseitig vorgesehene einseitige Verbindung mit dem Zwischenstück gewährleistet eine leichte Verformbarkeit des Verschlußkörpers relativ zum Zwischenstück, so daß sich der Verschlußkörper ohne weiteres an die Lage der Körperwand im Bereich der Körperöffnung anpassen kann. Dadurch wird die Fixierung des Okkluders in der Körperöffnung erleichtert und eine weitgehend vollständige Anlage der Verschlußkörper an die Körperwände gewährleistet.

Das Zwischenstück kann eine beispielsweise rechtwinklige Abstufung mit wenigstens zwei entgegengesetzt verlaufenden Schenkeln oder Schenkelpaaren aufweisen, wobei jeder Schenkel bzw. jedes Schenkelpaar an seinem freien Ende mit einem Verschlußkörper verbunden ist. Der Abstand zwischen den Vierschlußkörpern im Verschlußzustand wird dabei durch die Höhe der Abstufung festgelegt. Es versteht sich, daß die Abstufung oder Abwicklung auch einen Winkel von mehr oder weniger als 90° aufweisen kann, was das Anlegen der Verschlußkörper an die Körperwände und die Abdichtung der Körperöffnung erleichtert. Darüber hinaus kann das Zwischenstück auch eine Abbiegung oder eine Schlaufe aufweisen. Es kann ein S-förmiger Verlauf des Zwischenstücks vorgesehen sein oder auch ein gerader Verlauf des Zwischenstücks, wobei das Zwischenstück als Diagonalsteg zwischen den gegenüberliegenden Verschlußkörpem vorgesehen und mit den Verschlußkörpern auf gegenüberliegenden Seiten im Randbereich verbunden ist. Beide Schenkel bzw. Schenkelpaare des Zwischenstücks können die gleiche Länge aufweisen, so daß die Abstufung im Verschlußzustand im wesentlichen zentrisch zu den Verschlußkörpern angeordnet ist. Dadurch wird die Fixierung des Okkluders in der Körperöffnung verbessert, wobei das Zwischenstück im Bereich der Abstufung durch die Körperöffnung hindurchgeführt ist. Ist der Verschlußkörper ringförmig ausgebildet und umspannt eine kreisförmige (Verschluß-)Fläche, so kann die Länge des oder der mit einem Verschlußkörper verbundenen Schenkel des Zwischenstücks im wesentlichen dem Radius der kreisförmigen Fläche entsprechen.

Um eine weitgehend vollständige Anlage der Verschlußkörper gegen die Körperwände zu ermöglichen, ist vorzugsweise vorgesehen, daß die Verschlußkörper im wesentlichen in Richtung der Körperöffnung hintereinander angeordnet sind. Bei einer anderen Ausführungsform kann auch vorgesehen sein, daß die Verschlußkörper seitlich zueinander versetzt angeordnet sind. Der Überlappungsgrad der Verschlußkörper wird dabei beispielsweise durch die Länge der mit dem Verschlußkörper verbundenen Schenkel des Zwischenstücks festgelegt, so daß der Überlappungsgrad letztlich auch von der Form des Zwischenstücks abhängt. Dadurch kann der Okkluder in einfacher Weise an die Form der Körperwandungen angepaßt werden und bewirkt ein Anpressen beider Verschlußkörper gegen die Körperwände.

Der Okkluder bzw, die den Okkluder oder Teile des Okkluder bildende Leiterschleife ist vorzugsweise derart streckbar ausgebildet, daß der Okkluder in gestrecktem Zustand mittels Katheter implantiert werden und während der Implantation oder erst an seinem Zielort entfaltet werden kann. Dies erleichtert den Implantationsvorgang. In diesem Zusammenhang können die Verschlußkörper und das Zwischenstück aus einem einstückigen Draht aus einer Formgedächtnis-Legierung gebildet oder aus einem Rohr aus einer Formgedächtnis-Legierung geschnitten sein. Die endgültige Formgebung kann eine Wärmebehandlung erfordern. Die Verschlußkörper und das Zwischenstück bestehen im Ergebnis aus einem Materialstück. Dies ermöglicht eine einfache und kostengünstige Herstellung und vereinfacht die Implantation mittels Katheter. Der Verschlußkörper und das Zwischenstück können beispielsweise durch mehrfaches längsweises Schneiden eines Rohres, insbesondere eines Nitinol-Rohres, und anschließendes Aufdehnen hergestellt werden. Dabei bilden Rohrabschnitte die beiden gegenüberliegenden Verschlußkörper. Bildet der Okkluder einen elektrischen Resonanzschwingkreis, so kann ein zentraler Rohrabschnitt aufgetrennt werden, um eine Leiterschleife auszubilden und mittels einer elektrischen Isolation wieder zusammengefügt werden, um die Kapazität des Resonanzschwingkreises auszubilden. Hierauf wird nachfolgend noch im einzelnen eingegangen.

Durch die Verwendung einer Formgedächtnis-Legierung wird das Strecken des Okkluders in eine langgezogene Form zur Implantation mittels Katheter erleichtert. Beim Freigeben federt der Okkluder in die Verschlußstellung ein. Durch das Formgedächtnis wird eine sichere Fixierung des Okkluders in der Öffnung nach dem Entfalten der Verschlußkörper ermöglicht, wobei die Verschlußkörper aufgrund der durch das Formgedächtnis festgelegten Verformungskräfte gegen die Körperwandungen im Bereich der Körperöffnung angedrückt werden. Bilden die Verschlußkörper Spulen eines von dem Okkluder ausgebildeten Resonanzschwingkreises, so kann eine lokale Signalüberhöhung bereits nach einer Teilentfaltung des Okkluders zurückgegeben werden. Beispielsweise kann der proximal gelegene Verschlußkörper nach dem Entfalten im linken Atrium eine Signalantwort geben, wodurch die Implantation bzw. die Ausrichtung des Okkluders in der Körperöffnung wesentlich erleichtert werden kann.

Um eine sichere Fixierung des Okkluders in der Körperöffnung zu gewährleisten, ist vorgesehen, daß der Okkluder durch einen an seinen Enden zusammenlaufenden einstückigen Draht gebildet wird, wobei der Draht an entgegengesetzten Stellen jeweils zu einem einen außenliegenden Verschlußkörper bildenden Drahtring verformt ist, wobei der Drahtring zwei aufeinander zulaufende Drahtabschnitte aufweist, wobei die Drahtabschnitte radial in Richtung zum Mittelpunkt des Drahtrings abgebogen sind und in neben einander verlaufende Schenkel übergehen und wobei die Schenkel der beiden Verschlußkörper das innenliegende Zwischenstück bilden. Dies gewährleistet eine einfache Herstellung des Okkluders, wobei der Einfachheit halber die Schenkel der beiden Verschlußkörper im Bereich des Zwischenstücks, insbesondere im Bereich der Abstufung, miteinander verbunden sein können. Im Ergebnis weist das Zwischenstück auf beiden Seiten der Abstufung jeweils ein Schenkelpaar auf, das an dem jeweils freien Ende in den einen Verschlußkörper bildenden Drahtring übergeht. Die Schenkelpaare können im Bereich der Abstufung verlötet, verschweißt oder verklebt sein.

Die freien Enden des den Okkluder bildenden Drahtes können einen Kondensator des Resonanzschwingkreises ausbilden oder enthalten. Die zum Resonanzschwingkreis gehörende Kapazität kann in Form eines Plattenkondensators realisiert sein, wobei an den beiden freien Enden des Drahtes zwei gegenüberliegende Platten angeschlossen sein können. Alternativ kann die zum Resonanzschwingkreis gehörende Kapazität in Form von zwei dicht nebeneinander liegenden Drahtabschnitten realisiert werden, wobei die beiden freien Enden des Drahtes über eine vorgegebene Länge und einen vorgegebenen Abstand parallel nebeneinander geführt sind. Ebenso gut ist es möglich, daß die zum Resonanzschwingkreis gehörende Kapazität durch dicht gegenüberliegende Enden des Drahtes bzw. deren Querschnittsfläche gebildet wird, wobei die beiden gegenüberliegenden Drahtenden parallel und mit geringem Abstand zueinander angeordnet sein können. Es versteht sich, daß es darüber hinaus durch parallel verlaufende Drahtabschnitte zur Ausbildung parasitärer Kapazitäten kommen kann.

Der Verschlußkörper umspannt eine Verschlußfläche- bzw. ebene, wobei die das Zwischenstück bildenden Schenkel vorzugsweise außerhalb der Verschlußfläche verlaufend angeordnet sein können. Dadurch kann eine Störung der von dem Verschlußkörper ausgebildeten Induktivität weitgehend ausgeschlossen werden.

Wenigstens ein Verschlußkörper und/oder das Zwischenstück und/oder der Okkluder können mit einem Gewebe umhüllt bzw. ummantelt sein. Hier kann auf die aus dem Stand der Technik an sich bekannten Kunststoffgewebe, vorzugsweise ein Gewebe aus Polytetrafluorethylen oder Polyester oder ein unter dem Handelsnamen Dacron® erhältliches Gewebe, zurückgegriffen werden. Bei der Gewebeumhüllung kann es sich auch um ein Metallgewebe oder um ein Metallnetz handeln. Im übrigen ist auch eine Umhüllung mit einer Folie oder die Beschichtung mit einem Metalldünnfilm aus einem sehr gewebeverträglichen Metall möglich. Durch das Gewebe kann der Okkluder eine Filterfunktion erfüllen, wobei Thromben an dem Gewebe hängen bleiben. Zudem wird durch das Gewebe die Abdichtung der Körperöffnung verbessert und das Anwachsen von Körpergewerbe auf den Verschlußkörpern erleichtert. Schließlich kann durch die Art und Anordnung der Gewebeumhüllung Einfluß auf die Kapazität eines von dem Okkluder gebildeten Resonanzschwingkreises genommen werden.

Das Zwischenstück kann vorzugsweise im Bereich der Abstufung ummantelt sein, was die Implantation des Okkluders erleichtert und das Anwachsen von Haut verstärkt. Hier kann ein Gerüst aus einem biokompatiblen perforierten Kunststoff vorgesehen sein. Im übrigen kann die Ummantelung eine Führungsöffnung für einen Führungsdraht aufweisen, der das Einfädeln des Okkluders in die Körperöffnung beim Implantationsvorgang ermöglicht.

In einer weiteren vorteilhaften Ausführungsform weist der Okkluder wenigstens einen haken- oder ösenförmigen Angriffsabschnitt für ein Implantationswerkzeug auf. Der Angriffsabschnitt kann durch einen als Schenkelfeder oder als Öse ausgebildeten Abschnitt eines den Okkluder bildenden Drahtstücks vorgesehen sein oder es können gesonderte Bauelemente eingesetzt werden, die mit dem Okkluder verbunden sind. Der Angriffsabschnitt ermöglicht die Kopplung des Okkluders mit einem Werkzeug zum Implantieren im gestreckten Zustand mittels Katheter oder zum Entfalten des Okkluders. Dies ermöglicht dem Operateur eine leichte Handhabung des Okkluders. Darüber hinaus kann der Angriffsabschnitt als Führung für einen Führungsdraht vorgesehen sein, mit dem der Okkluder in die Körperöffnung eingefädelt wird. Der Angriffsabschnitt kann auch zum Zurückziehen des teil- oder vollentfalteten Okkluders, beispielsweise beim Bergen des Okkluders, vorgesehen sein.

Das den Okkluder bildende Drahtstück kann an Knick- oder Biegestellen als Schenkelfeder ausgebildet sein, was zu einer hohen Formstabilität des erfindungsgemäßen Okkluders beiträgt. Die Schenkelfedern können darüber hinaus das Strecken und Entfalten des Okkluders vereinfachen.

Darüber hinaus läßt es die Erfindung zu, daß das Zwischenstück ebenfalls eine Abdicht- oder Zentrierfunktion beim Verschluß der Körperöffnung erfüllt. In diesem Zusammenhang ist vorgesehen, daß das Zwischenstück eine zum Abdichten der Körperöffnung und/oder zum Zentrieren und/oder zum Verankern des Okkluders in der Körperöffnung ausgebildete Form aufweist. Dies kann beispielsweise bedeuten, daß das Zwischenstück wenigstens eine Schenkelfeder aufweist, wobei, vorzugsweise, von der Schenkelfeder eine weitere Verschlußfläche aufgespannt wird, die im wesentlichen parallel zu einer von einem Verschlußkörper als solchem aufgespannten Verschlußfläche und damit im wesentlichen in der Ebene der defekten Wandteile angeordnet ist. Im Implantationszustand ist dann die Schenkelfeder des Zwischenstücks innerhalb der Körperöffnung angeordnet, wobei der Okkluder durch die Schenkelfeder in der Körperöffnung verankert und ggf. zentriert wird. Darüber hinaus kann die Schenkelfeder eine Abdichtfunktion verfüllen. Weitere Vorteile ergeben sich, wenn das Zwischenstück Mittel aufweist, die die Verankerung in der Körperöffnung erleichtern, beispielsweise eine außen liegende Verzahnung. Durch eine geeignete Formgebung des Zwischenstücks kann sichergestellt werden, daß die vorgenannten Funktionen erfüllt werden. Beispielsweise kann sich das Zwischenstück in Längsrichtung der Körperöffnung erstrecken und eine Taillierung in der Mitte aufweisen, die ebenfalls zu einer Zentrierung des Okkluders in der Körperöffnung führt. Das Zwischenstück kann die Funktion eines weiteren Verschlußkörpers erfüllen, der mit den vor und hinter dem Wanddefekt angeordneten Verschlußkörpern des Okkluders zusammenwirkt, um den möglichst vollständigen Verschluß eines Septum- oder Gefäßdefektes zu bewirken bzw. zu unterstützen.

Um den Okkluder deutlich und signalintensiv im MR-Bild darstellen zu können, ist vorgesehen, daß der Okkluder oder Teile des Okkluders einen elektrischen Resonanzschwingkreis ausbilden, wobei wenigstens eine die Induktivität des Resonanzschwingkreises ausbildende Leiterschleife vorgesehen ist und der Okkluder oder Teile des Okkluders von der Leiterschleife ausgebildet werden. Erfindungsgemäß ist vorgesehen, nur eine Struktur, nämlich eine Leiterschleife, sowohl für die Ausbildung des eigentlichen Okkluders als auch für die Induktivität zu verwenden. In Kombination mit einer Kapazität wird somit ein Resonanzschwingkreis bereitgestellt. Ein Okkluder, der als Resonanzschwingkreis ausgebildet ist, verbessert die Visualisierung des Implantats in einem MR-Bild, wobei, vorzugsweise, die Resonanzfrequenz des Schwingkreises im wesentlichen gleich der Resonanzfrequenz der eingestrahlten RF-Strahlung des MR-Bildgebungssystems ist. Dies trägt zu einer deutlichen Verringerung von RF-Artefakten bei. Im Ergebnis kann die Implantation des erfindungsgemäßen Okkluders ohne weiteres und ohne zwingenden Einsatz eines Röntgenverfahrens mit einem MR-Verfahren überwacht werden. Der Okkluder weist erfindungsgemäß wenigstens einen geschlossenen Schwingkreis mit einer Induktivität und einer Kapazität auf, wobei dieses System eine verändernde Signalantwort erzeugt, das von wenigstens einer Empfangsspule detektierbar und ortsaufgelöst darstellbar ist.

Der erfindungsgemäße Okkluder ist vorzugsweise zum Verschluß von Septum - oder Gefäßdefekten vorgesehen. Es versteht sich, daß der Okkluder grundsätzlich auch zum Verschluß von beliebigen Wanddefekten in Hohlorganen des menschlichen oder tierischen Körpers einsetzbar ist. Insbesondere ist der erfindungsgemäße Okkluder einsetzbar zum Verschluß eines persistierenden Atriumseptumdefektes, insbesondere eines persistierenden Foramen ovale (PFO) oder eines Atriumseptumdefektes vom Sekundum-Typ (ASD II). Auch Ventrikelseptumdefekte (VSD) können mit dem erfindungsgemäßen Okkluder verschlossen werden. Schließlich ist der Okkluder beispielsweise auch einsetzbar, um Fisteln zu verschließen. Im übrigen kann der Okkluder zum Verschluß eines persistierenden Ductus arteriosus (Ductus-Botalli) vorgesehen sein.

Bei der erfindungsgemäßen Ausführungsform können die Verschlußkörper mit einem Grewebeüberzug, einer (Metall-)Beschichtung oder einer Folie ausgerüstet sein, um eine Abdichtung der Körperöffnung durch Stimulation des Gewebewachstums im Inneren der Körperöffnung bzw. im Kanal des Defektes zu unterstützen und/oder eine Filterfunktion zu erfüllen.

Unter einem "Verschlußkörper" im Sinne der Erfindung wird bei der vorgenannten Ausführungsform ein vorzugsweise schirmförmiges, ringförmiges, scheibenförmiges oder auch blumenartiges (Flächen-)Element bzw. ein Rahmenteil verstanden, das im Verschlußzustand auf einer Seite der Öffnung gegen die die Körperöffnung umgebenden (defekten) Körperwände anliegt. Es versteht sich, daß der Verschlußkörper grundsätzlich auch andere Querschnittsformen aufweisen kann. Im übrigen kann sich der Verschlußkörper aus mehreren Segmenten zusammensetzen, wobei ein Verschlußkörper bei der ersten Ausführungsform jeweils die Gesamtheit aller auf einer Wandseite vorgesehenen Segmente umfaßt.

Bei einer Ausführungsform der Erfindung bildet der Okkluder einen oder mehrere Resonanzschwingkreise mit jeweils wenigstens einer Leiterschleife aus. Dadurch kann beispielsweise erreicht werden, daß der Okkluder mit mehreren, unterschiedlichen MR-Frequenzen betrieben und detektiert werden kann. Auch kann vorgesehen sein, daß mehrere Resonanzschwingkreise miteinander gekoppelt sind. Darüber hinaus kann die Leiterschleife mit einem Nichtleiter, insbesondere Kunststoff und/oder Keramik, ummantelt sein. Dies dient einer vergrößerten Haltbarkeit durch vergrößerte mechanische Stabilität und einer störungsfreien Funktion des Okkluders. In einer besonders bevorzugten Ausführungsform kann die Isolierung zur Verminderung und Regulierung der parasitären Kapazität vorgesehen sein, wobei die Isolierung auch für die Feineinstellung der Resonanzfrequenz verwendet werden kann. Die Isolierungsschicht bzw. die Ummantelung kann in Verbindung mit wenigstens einer Leiterschleife gleichzeitig eine interne Kapazität ausbilden.

Vorzugsweise hat der Resonanzschwingkreis eine Resonanzfrequenz, insbesondere im hochfrequenten Bereich, die der Frequenz eines äußeren Magnetfeldes, insbesondere der Larmor-Frequenz eines MR-Tomographen, entspricht. Damit wird gewährleistet, daß der erfindungsgemäße Okkluder in einem MR-Bildgebungssystem gut darstellbar ist, was es ermöglicht, die Implantation und auch die Verschlußfunktion des Okkluders in einfacher Weise zu überwachen. Es versteht sich, daß der Schwingkreis grundsätzlich auch eine Resonanzfrequenz in einem anderen Frequenzbereich aufweisen kann.

Die Leiterschleife kann wenigstens ein elektrisch nicht-leitendes Material aufweisen, auf dessen Oberfläche wenigstens ein leitendes Material, insbesondere Gold, Platin, Tantal und/oder eine leitfähige Legierung, aufgebracht sein kann. Die Beschichtung des Okkluders mit einem besonders leitfähigen Material, wie z.B. Gold, verbessert die Ausbildung der Resonanz. Anstelle von Gold können auch Platin oder Tantal zur Beschichtung des Okkluders eingesetzt werden, wobei Tantal eine hohe elektrochemische Kompatibilität aufweist. Es versteht sich, daß auch mehrere Schichten aus Isolator und Leiter auf die Leiterschleife aufgebracht sein können. Auf dem Okkluder kann eine dünne Schicht aus einem Haftvermittler aufgetragen sein, der die Haftung des elektrisch leitenden Materials auf dem Okkluder verbessert. Bei einer weiteren Ausführungsform kann vorgesehen sein, daß zur Ausbildung einer Induktivität des Schwingkreises eine selektive Beschichtung des Okkluders mit einem elektrisch leitenden Material, insbesondere mit Gold oder Platin, vorgesehen ist.

Wie bereits eingangs darauf hingewiesen worden ist, kann der erfindungsgemäße Okkluder mehrere Leiterschleifen aufweisen. Dies ermöglicht eine größere Flexibilität bei der Ausgestaltung des Okkluders und kann die Resonanz weiter verbessern. Die Leiterschleifen können elektrisch leitend miteinander verbunden sein. In diesem Zusammenhang wird unter dem Begriff "Leiterschleife" ein aus einem Stück bestehender Leiter verstanden.

Erfindungsgemäß kann vorgesehen sein, daß wenigstens ein Verschlußkörper von der Leiterschleife gebildet wird. Vorzugsweise wird der Okkluder von einer Leiterschleife gebildet, die die Induktivität des Schwingkreises ausbildet.

Darüber hinaus ist es vorzugsweise vorgesehen, daß die Leiterschleife eine Kapazität des Resonanzschwingkreises ausbildet. Beispielsweise kann ein Kondensator durch parallel verlaufende Abschnitte der Leiterschleife verwirklicht sein. Alternativ ist es grundsätzlich natürlich auch möglich, daß der Kondensator durch ein gesondertes Bauelement, wie beispielsweise ein SMD-Kondensator, der in den Okkluder integriert ist, verwirklicht ist.

Im einzelnen gibt es eine Vielzahl von Möglichkeiten, den erfindungsgemäßen Okkluder auszugestalten und weiterzubilden, wobei einerseits auf die abhängigen Patentansprüche und andererseits auf die nachfolgende detaillierte Beschreibung eines bevorzugten Ausführungsbeispiels der Erfindung unter Bezugnahme auf die Zeichnung verwiesen wird. Im übrigen läßt es die Erfindung bedarfsweise zu, die in den Ansprüchen genannten und/oder die anhand der Zeichnung offenbarten und beschriebenen Merkmale miteinander zu kombinieren, auch wenn dies nicht im einzelnen beschrieben ist. In der Zeichnung zeigt:
- Fig. 1: eine schematische Querschnittsansicht eines mit einem Okkluder verschlossenen Atriumseptumdefektes des menschlichen Herzens,
- Fig. 2: ein mit einem erfindungsgemäßen Okkluder verschlossener Wanddefekt in einer schematischen Querschnittsansicht,
- Fig. 3: eine perspektivische Darstellung eines erfindungsgemäßen Okkluders im entfalteten Zustand,
- Fig. 4: eine schematische Draufsicht auf eine zweite Ausführungsform eines erfindungsgemäßen Okkluders,
- Fig. 5: eine schematische Draufsicht auf eine dritte Ausführungsform eines erfindungsgemäßen Okkluders,
- Fig. 6: eine schematische Draufsicht auf eine vierte Ausführungsform eines erfindungsgemäßen Okkluders,
- Fig. 7: eine schematische Draufsicht auf eine fünfte Ausführungsform eines erfindungsgemäßen Okkluders,
- Fig. 8: eine erste Ausführungsform einer eine Kapazität bildenden Leiterschleife eines Okkluders in einer Teilschnittansicht,
- Fig. 9: eine weitere Ausführungsform einer eine Kapazität bildenden Leiterschleife eines Okkluders in einer Teilschnittansicht,
- Fig. 10: der in Fig. 3 dargestellte Okkluder in einem langgestreckten Zustand,
- Fig. 11: eine schematische Darstellung von kapazitätsbildenden Flächen des Zwischenteils eines Okkluders,
- Fig. 12-14: mögliche Schnittmuster eines Rohres zur Herstellung einer Leiterschleife eines Okkluders,
- Fig. 15-22: eine schematische Darstellung des Implantationsvorgangs eines Okkluders zum Verschluß des in Fig. 2 dargestellten Wanddefektes und
- Fig. 23-27: eine andere Ausführungsform eines Okkluders.

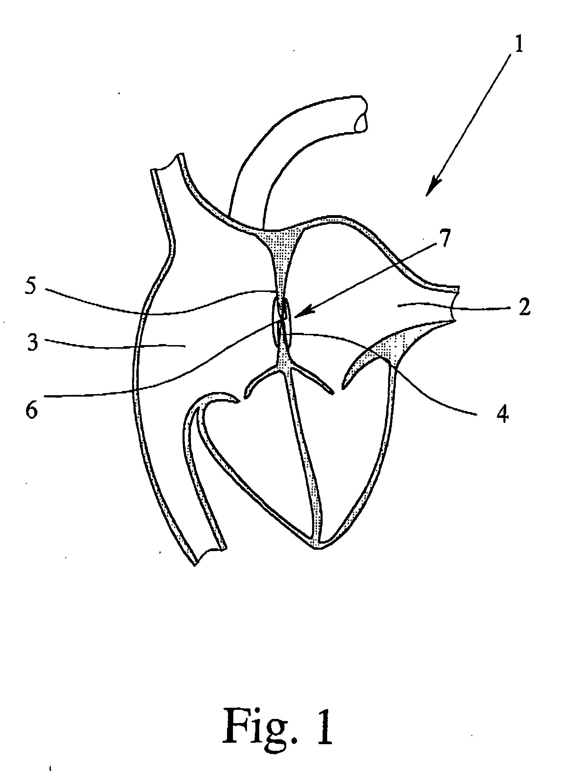

In Fig. 1 ist ein Atriumseptumdefekt (ASD) eines menschlichen Herzens 1 dargestellt, wobei das Herz 1 einen linken Vorhof 2 und einen rechten Vorhof 3 aufweist. Der linke Vorhof 2 und der rechte Vorhof 3 werden durch das Septum primum 4 und das Septum secundum 5 voneinander getrennt. Zwischen dem Septum primum 4 und dem Septum secundum 5 ist eine Körperöffnung 6, das sogenannte Foramen ovale, dargestellt. Im Regelfall schließt sich die Öffnung 6 in den ersten Lebensmonaten. Bei etwa 20% der Menschen allerdings erfolgt der Verschluß unvollständig. Dieses persistierende Foramen ovale hat keine Auswirkungen auf das Befinden und wird von seinem Träger in der Regel nicht wahrgenommen, bleibt jedoch ggf. nicht gänzlich folgenlos. Unter Umständen kann der Eintritt eines Schlaganfalls begünstigt werden. Zum Verschluß der Körner-öffnung 6, bei der es sich allgemein um einen Septum- oder Gefäßdefekt handeIn kann, kann ein Okkluder 7 eingesetzt werden, der mittels Katheter im Bereich der Öffnung 6 platziert wird.

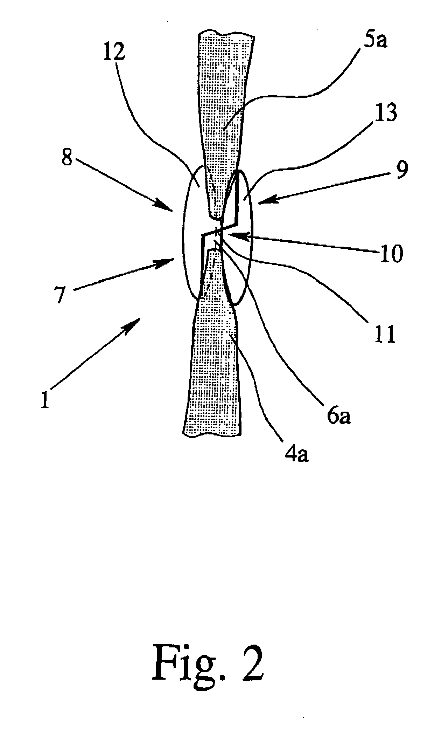

In Fig. 2 ist ein Wanddefekt einer Körperhöhle mit defekten Wandteilen 4a, 5a dargestellt, bei dem eine Öffnung 6a mit einem Okkluder 7 verschlossen ist. Der Okkluder 7 weist im Verschlußzustand der Körperöffnung 6a auf gegenüberliegenden Seiten der Körperöffnung 6a angeordnete zumindest bereichsweise gegenüberliegende ringförmige Verschlußkörper 8, 9 auf. Die Verschlußkörper 8, 9 sind mit einem Zwischenstück 10 miteinander verbunden, wobei das Zwischenstück 10 im Bereich einer Abstufung 11 durch die Körperöffnung 6a hindurchgeführt ist. Der Okkluder 7 liegt bei der dargestellten Ausführungsform mit den ringförmigen Verschlußkörpern 8, 9 gegen die Körperwandungen an, die nach dem Implantieren des Okkluders 7 mit Gewebe überwachsen, was zu einem vollständigen Verschluß der Körperöffnung 6a führt. Die Verschlußkörper 8, 9 umrahmen Verschlußflächen 12, 13 und können mit einem Gewebeüberzug umhüllt sein, um das Anwachsen von Gewebe zu erleichtern.

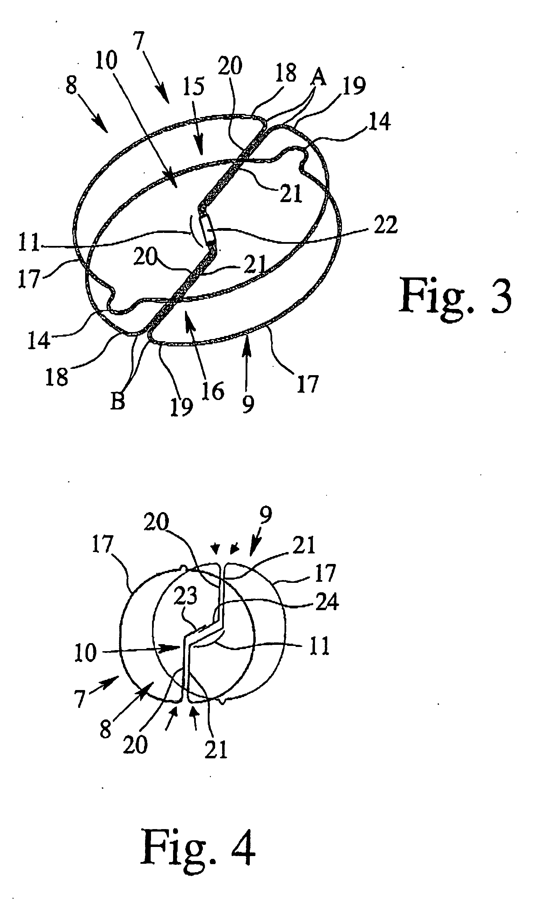

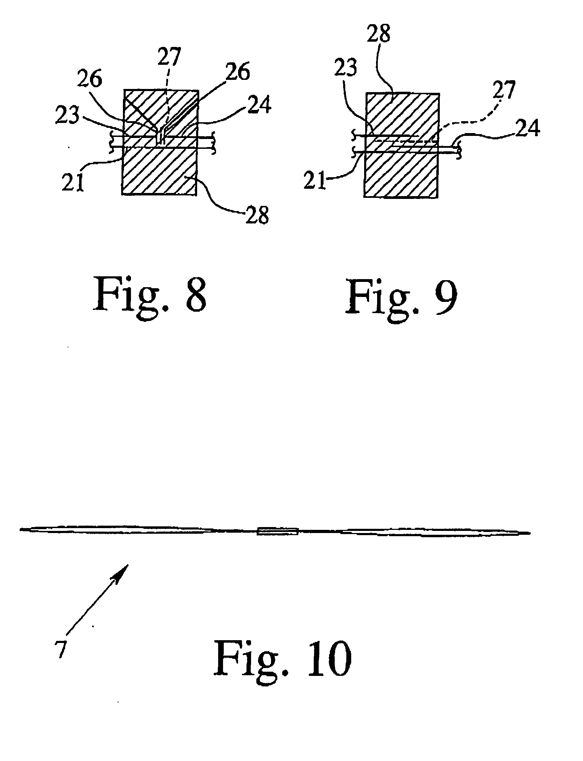

In Fig. 3 ist eine bevorzugte Ausführungsform des erfindungsgemäßen Okkluders 7 perspektivisch dargestellt. Wie sich aus einem Vergleich der Fig. 3 und 10 ergibt, läßt sich der Okkluder 7 durch Strecken in eine langgestreckte Form überführen, die das einfache Implantieren des Okkluders 7 mit einem Katheter ermöglicht. Um den Okkluder in den gestreckten Zustand zu führen bzw. den Okkluder 7 zusammenzulegen, sind hakenförmige Angriffsabschnitte 14 an beiden Verschlußkörpern 8, 9 vorgesehen.

Der in Fig. 3 dargestellte Okkluder 7 weist eine einseitige Verbindung zwischen dem Zwischenstück 10 und dem Verschlußkörper 8, 9 auf, wobei das Zwischenstück 10 im Verschlußzustand mit jedem Verschlußkörper 8, 9 exzentrisch im Randbereich des Verschlußkörpers 8, 9 verbunden ist und wobei die im Verschlußzustand auf gegenüberliegenden Seiten der Körperöffnung 6 angeordneten gegenüberliegenden Verschlußkörper 8, 9 auf entgegengesetzten Seiten A, B mit dem Zwischenstück 10 verbunden sind. Dadurch wird gewährleistet, daß der Okkluder 7 nach der Implantation mit beiden Verschlußkörpern 8, 9 im wesentlichen vollständig gegen die die Körperöffnung 6 umgebenden Körperwände anliegt. Die einseitige Befestigung der Verschlußkörper 8, 9 mit dem Zwischenstück 10 ermöglicht es, daß sich der Verschlußkörper 8, 9 an die körperspezifische Ausbildung der Körperwände sehr gut anpaßt.

Das Zwischenstück 10 weist eine Abstufung 11 mit zwei entgegengesetzt verlaufenden Schenkelpaaren 15, 16 auf, wobei jedes Schenkelpaar 15, 16 mit seinem freien Ende mit einem Verschlußkörper 8, 9 verbunden ist. Die Schenkelpaare 15, 16 des Zwischenstücks 10 weisen dieselbe Länge auf, so daß die Abstufung 11 im Verschlußzustand im wesentlichen zentrisch zu den auf gegenüberliegenden Seiten der Körperöffnung 6 angeordneten Verschlußkörpern 8, 9 angeordnet ist. Dadurch wird die sichere Anlage der Verschlußkörper 8, 9 gegen die Körperwände erleichtert und die Fixierung des Okkluders 7 in der Körperöffnung 6 gewährleistet.

Bei der in Fig. 3 dargestellten Ausführungsform wird der Okkluder durch einen an seinen Enden zusammenlaufenden einstückigen Draht 17 gebildet, wobei der Draht 17 an entgegengesetzten Stellen jeweils zu einem einen außenliegenden Verschlußkörper 8, 9 bildenden Drahtring verformt ist. Der Drahtring weist zwei aufeinander zulaufende Drahtabschnitte 18, 19, wobei die Drahtabschnitte 18, 19 radial in Richtung zum Mittelpunkt des Drahtrings abgebogen sind und in parallel zueinander verlaufende gemeinsame Schenkel 20, 21 übergehen. Die Schenkel 20, 21 bilden zu beiden Seiten der Abstufung 11 die Schenkelpaare 15, 16 aus. Das Zwischenstück 10 umfaßt somit die Schenkelpaare 15, 16 und die Abstufung 11.

Bei dem in Fig. 3 dargestellten Okkluder 7 sind die Schenkel 20, 21 der beiden Verschlußkörper 8, 9 im Bereich der Abstufung 11 elektrisch leitend miteinander verbunden, wobei die Schenkel 20, 21 durch eine Keramikhülse 22 fest verbunden sind. Dadurch wird eine formstabile Anordnung des Okkluders 7 sichergestellt. Die Schenkel 20, 21 sind allerdings mit Ausnahme der Abstufung 11 nicht verbunden und biegbar, so daß der von dem Draht 17 gebildete ringförmige Verschlußkörper 8, 9 einfach an eine umgebende Körperwandung anpaßbar ist.

In den Fig. 4 bis 7 ist jeweils ein Okkluder 7 dargestellt, der einen elektrischen Resonanzschwingkreis ausbildet. Der Okkluder 7 wird wiederum jeweils durch einen einstückigen Draht 17 gebildet, wobei der Draht 17 eine Leiterschleife des Resonanzschwingkreises darstellt und eine Induktivität ausbildet. Die Leiterschleife weist zwei Windungen in Form der als Drahtring ausgebildeten Verschlußkörper 8, 9 auf. Darüber hinaus bildet die Leiterschleife bzw. der Draht 17 eine Kapazität des Resonanzschwingkreises aus, wobei gemäß der in den Fig. 4 bis 7 dargestellten Ausführungsformen die Drahtenden 23, 24 des Drahtes 17 über eine vorgegebene Strecke parallel nebeneinander geführt und voneinander beabstandet angeordnet sind. Zwischen den Drahtenden 23, 24 ist ein Dielektrikum ausgebildet. Beispielsweise kann vorgesehen sein, daß die Drahtenden 23, 24 mit einem nicht-leitenden Klebstoff verklebt sind.

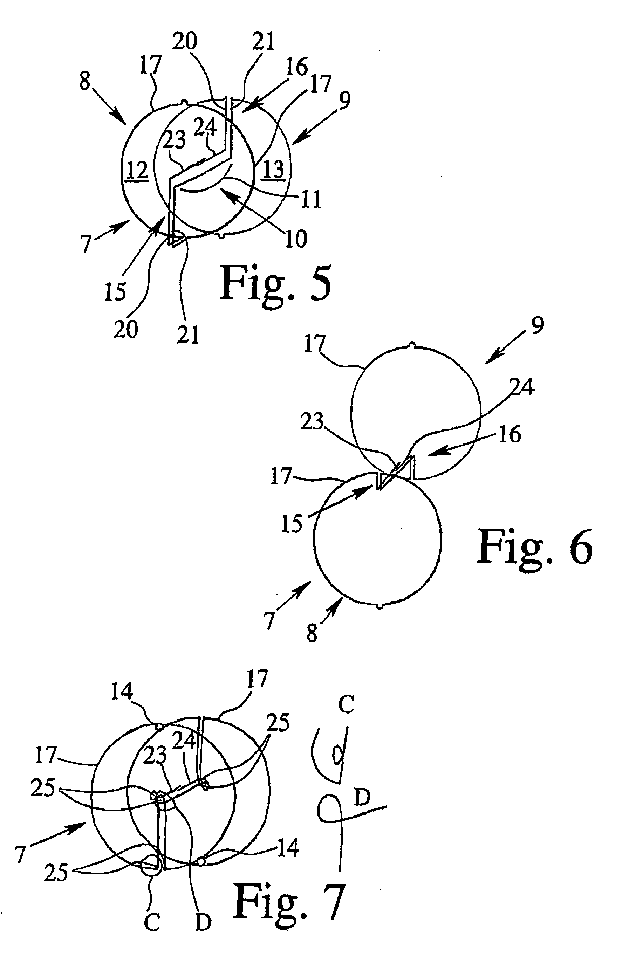

Bei dem in Fig. 3 dargestellten Okkluder 7 ist die Abstufung 11 des Zwischenstücks 10 im wesentlichen rechtwinklig zu den Schenkelpaaren 15, 16 angeordnet. Bei der in Fig. 6 dargestellten Ausführungsform weist der Okkluder 7 dagegen ein Zwischenstück 10 mit einer schräg gestellten Abstufung 11 auf. Dadurch wird eine vollständige Abdichtung der Körperöffnung 6 nach dem Einsetzen des Okkluders 7 durch die Verschlußkörper 8, 9 erleichtert.

In Fig. 5 ist eine Ausführungsform eines Okkluders 7 dargestellt, bei der die Schenkel 20, 21 der Schenkelpaare 15, 16 außerhalb der Verschlußflächen 12, 13 verlaufend angeordnet sind. Dazu ist vorgesehen, daß die den Drahtring des Verschlußkörpers 8, 9 bildenden und aufeinander zulaufenden Drahtabschnitte 18, 19 in axialer Richtung abgewinkelt und außerhalb der Verschlußfläche 12, 13 in Richtung zum Mittelpunkt des Drahtrings abgebogen sind. Die Abstufung 11 ist dabei durch die Verschlußflächen 12, 13 der Verschlußkörper 8, 9 hindurchgeführt. Ebenso gut kann vorgesehen sein, daß die Schenkel 20, 21 gegenüber den Verschlußflächen 12, 13 abgewinkelt sind, so daß die Schenkel 20, 21 zu keiner Störung der Induktivität des Resonanzschwingkreises führen können.

In Fig. 6 ist eine Ausführungsform eines Okkluders 7 dargestellt, bei der die Verschlußkörper 8, 9 quer zur Verschlußrichtung gegeneinander versetzt sind. Diese asymmetrische Anordnung der Verschlußkörper 8, 9 ermöglicht eine einfache Abdichtung der Körperöffnung 6, wobei die Verschlußkörper 8, 9 nur zu einem geringen Teil einander überdecken. Dies ist jedoch ausreichend, um den Verschluß der Körperöffnung 6 zu gewährleisten. Der Überlappungsgrad der Verschlußkörper 8, 9 wird dabei festgelegt durch die Länge der Schenkelpaare 15, 16.

In Fig. 7 ist dargestellt, daß der Okkluder 7 eine Mehrzahl von Schenkelfedern 25 aufweisen kann, die das Zusammenlegen des Okkluders 7 durch Strecken in Längsrichtung erleichtern und als Angriffsabschnitt für ein Implantationswerkzeug dienen können. Darüber hinaus tragen die Schenkelfedern 25 zu einer hohen Formstabilität des Okkluders 7 bei. Nicht dargestellt ist, daß eine Schenkelfeder 25 auch im Bereich der Abstufung 11 des Zwischenstücks 10 vorgesehen sein kann. Dabei kann vorzugsweise die von der Schenkelfeder 25 umrahmte Schenkelfläche parallel zu den von den Verschlußkörper 8,9 umrahmten Verschlußflächen angeordnet sein. In diesem Fall kann die Schenkelfeder 25 zur Verankerung des Okkluders 7 in dem Defekt beitragen. Im übrigen kann das Zwischenstück 10 eine beliebige Formgebung aufweisen, beispielsweise eine Taillierung im mittleren Bereich, um die Zentrierung des Okkluders 7 im Defekt zu verbessern. Auch kann das Zwischenstück 10 ausgebildet sein, um zur Abdichtung des Defekts beizutragen. Zur Verankerung mit den defekten Wandteilen im Bereich der Öffnung 6, 6a kann das Zwischenstück 10 bei geeigneter Formgebung eine außen liegende Verzahnung aufweisen, die mit den defekten Wandteilen im implantierten Zustand des Okkluders 7 zusammenwirkt. Im übrigen weist der in Fig. 7 dargestellte Okkluder 7 einen ösenförmigen Angriffsabschnitt 14 für einen Führungsdraht auf, der das Einfädeln des Okkluders 7 in eine Körperöffnung 6 erleichtert.

In den Fig. 8 und 9 ist ausschnittsweise dargestellt, daß die Leiterschleife des Okkluders 7 eine Kapazität des Resonanzschwingkreises ausbilden kann. Gemäß Fig. 8 sind im Bereich der Abstufung 11 an den Drahtenden 23, 24 gegenüberliegende Plättchen 26 angeschlossen, so daß die zum Resonanzschwingkreis gehörende Kapazität in Form eines Plattenkondensators realisiert wird. Zwischen den Plättchen 26 ist ein dielektrischer Bereich 27 vorgesehen. Die Drahtenden 23, 24 und der aus dem Draht 17 gebildete Schenkel 21 sind von einer Kunststoffhülse 28 umgeben. Nicht dargestellt ist, daß die Kunststoffhülse 28 eine Führungsöffnung für einen Führungsdraht aufweisen kann. Bei der in Fig. 9 dargestellten Ausführungsform ist schematisch dargestellt, daß eine Kapazität des durch den Okkluder 7 gebildeten Resonanzschwingkreises durch bereichsweise parallel nebeneinander geführte Drahtenden 23, 24 ausgebildet sein kann. Zwischen den Drahtenden 23, 24 ist wiederum ein dielektrischer Bereich 27 vorgesehen.

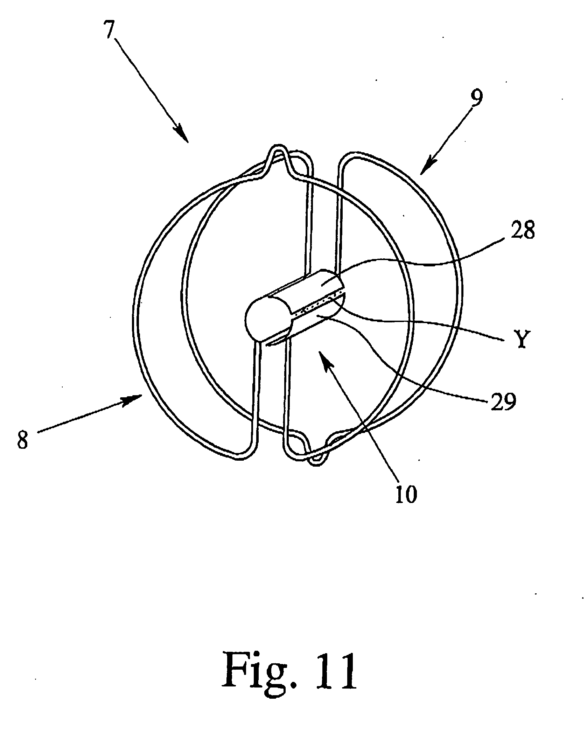

Der Okkluder 7 kann durch mehrfaches längsweises Schneiden eines Rohres, insbesondere eines Nitinol-Rohres, und anschließendes Aufdehnen hergestellt sein. In Fig. 11 ist ein Ausführungsbeispiel eines vollständig aus einem Nitinol-Rohr geschnitten Okkluders 7 nach dem Aufbiegen dargestellt, wobei durch geeignete Formgebung des Zwischenstücks 10 eine Kapazität zwischen den Teilflächen 28, 29 des Zwischenstücks 10 realisiert werden kann. Die zu realisierende Kapazität ist durch eine punktierte Linie Y schematisch dargestellt.

Nicht dargestellt ist, daß bei der Herstellung des Okkluders 7 die den Okkluder 7 bildenden Leiterabschnitte im Bereich des Zwischenstücks 10 zunächst durch Brücken miteinander verbunden sein können, um die Leiterstücke im Bereich des Zwischenstücks 10 mit einem bestimmten Abstand zueinander zu fixieren. Anschließend werden die Leiterstücke im Bereich des Zwischenstücks 10 in einer Einbettmasse eingebettet. Nach dem Aushärten der Einbettmasse werden die Brücken dann unterbrochen, wobei die Leiterstücke im eingebetteten Zustand einen definierten Abstand zur Ausbildung einer Kapazität aufweisen.

Der Okkluder 7 wird im Bereich des Zwischenstücks 10 anschließend in einer Einbettmasse eingebettet. Dadurch werden die Leiterstücke im Bereich des Zwischenstücks 10 relativ zueinander fixiert, so daß die Brücken zwischen den Leiterstücken dann gelöst werden können. Anschließend ist bei der Herstellung des Okkluders 7 die Erwärmung und die Verformung der Leiterstücke vorgesehen. Die Brücken stellen sicher, daß

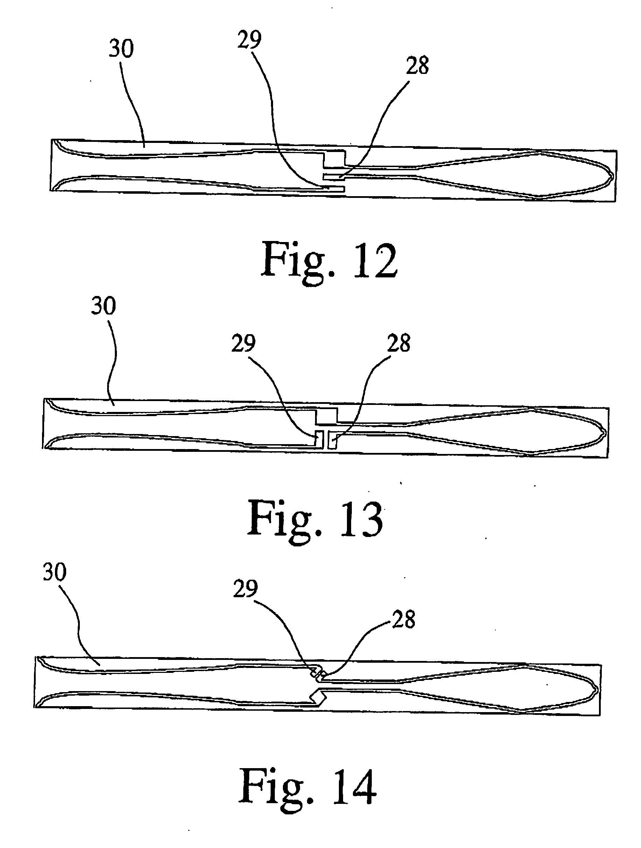

In den Figuren 12 bis 14 sind Schnittmuster eines Rohres 30 für alternative Ausführungsformen eines Okkluders 7 dargestellt, mit im Bereich des Zwischenstücks 10 vorgesehenen und unterschiedlich angeordneten kapazitätsbildenden Flächen 28, 29. Vorzugsweise handelt es sich bei dem Rohr 30 um ein Nitinol-Rohr mit einem Außendurchmesser von 1 bis 3 mm, insbesondere von 2 mm, und einer Wandstärke von 0,4 bis 0,6 mm, insbesondere von 0,2 mm. Das aus dem Rohr 30 ausgeschnittene Leiterstück wird vorzugsweise vergoldet, wobei die zwischen den kapazitätsbildenden Flächen 28, 29 vorgesehene Spalte bei dem Vergoldungsvorgang nicht verschlossen werden dürfen. Im Bereich des Zwischenstücks 10 kann der Okkluder 7 mittels Kunststoff, beispielsweise Epoxydharz, fixiert werden, so daß ein stabiler Ring entsteht, der eine unveränderliche Kapazität bildet und eventuell als Führung für einen Führungsdraht verwendet werden kann.

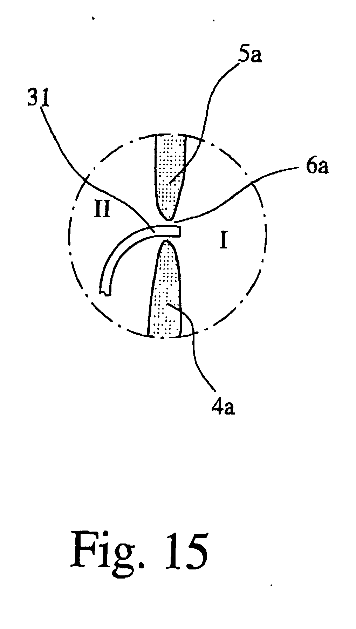

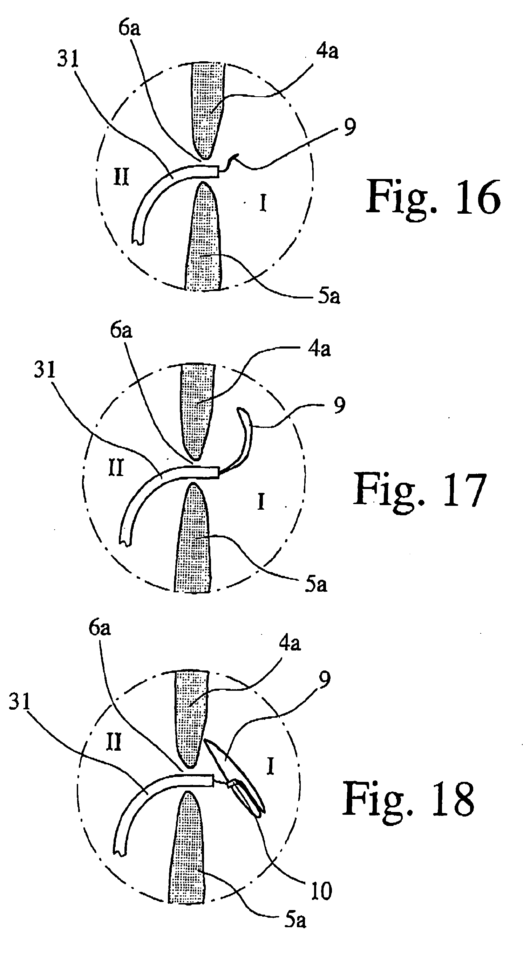

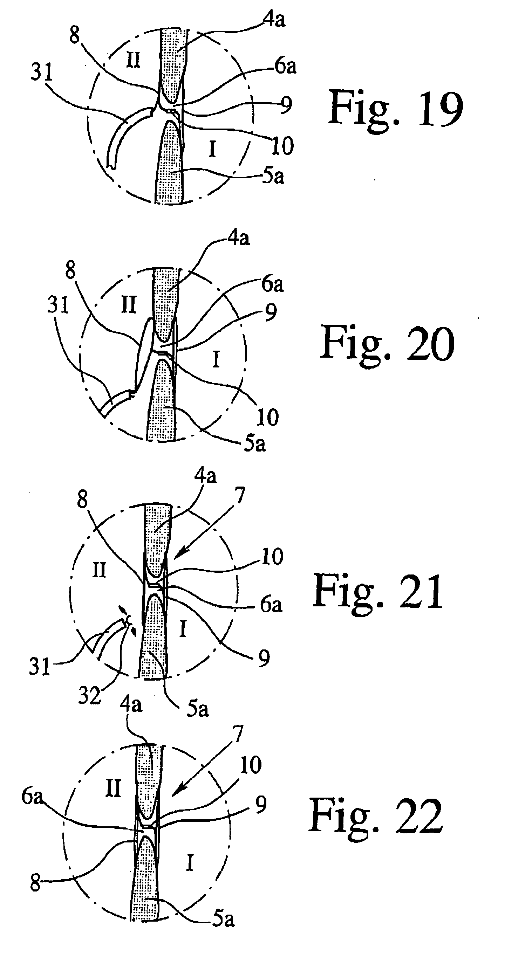

Anhand der Fig. 15 bis 22 wird schematisch der Implantationsvorgang eines Okkluders 7 in die in Fig. 2 dargestellte Körperöffnung 6a erläutert, wobei die Körperöffnung 6a von zwei defekten Wandteilen 4a, 5a begrenzt wird. Der Okkluder 7 wird durch einen Katheter 31 eingeführt, wobei das freie Ende des Katheters 31 durch die Körperöffnung 6a hindurch auf die eine Seite I des Wanddefektes positioniert wird. Die Fig. 16 bis 19 zeigen die Freisetzung und die Entfaltung des proximalen Verschlußkörpers 9 auf der Seite I des Wanddefektes. Fig. 19 zeigt das Anlegen des Verschlußkörpers 9 an die defekten Wandteile 4a, 5a auf der Seite I des Wanddefektes und das Freisetzen des Zwischenstücks 10 sowie den durch Zurückziehen des Katheters 31 zum Teil freigesetzten distalen Verschlußkörper 8 des Okkluders 7 auf der anderen Seite II der Körperöffnung 6a. Durch weiteres Zurückziehen des Katheters 31 kommt es zur vollständigen Freisetzung des distalen Verschlußkörpers 8, was in den Fig. 20 und 21 dargestellt ist. Durch Öffnen einer Greifeinrichtung 32 des Katheters 31 kommt es zur Freisetzung des Okkluders 7, wobei in Fig. 22 der Okkluder 7 im vollständig entfalteten Zustand dargestellt ist. Die Verschlußkörper 8, 9 werden über das Zwischenstück 10 auf beiden Seiten I, II des Wanddefektes gegen die defekten Wandteile 4a, 5a gedrückt, was zu einem Verschluß der Körperöffnung 6a durch Gewebewachstum in diesem Bereich führt.

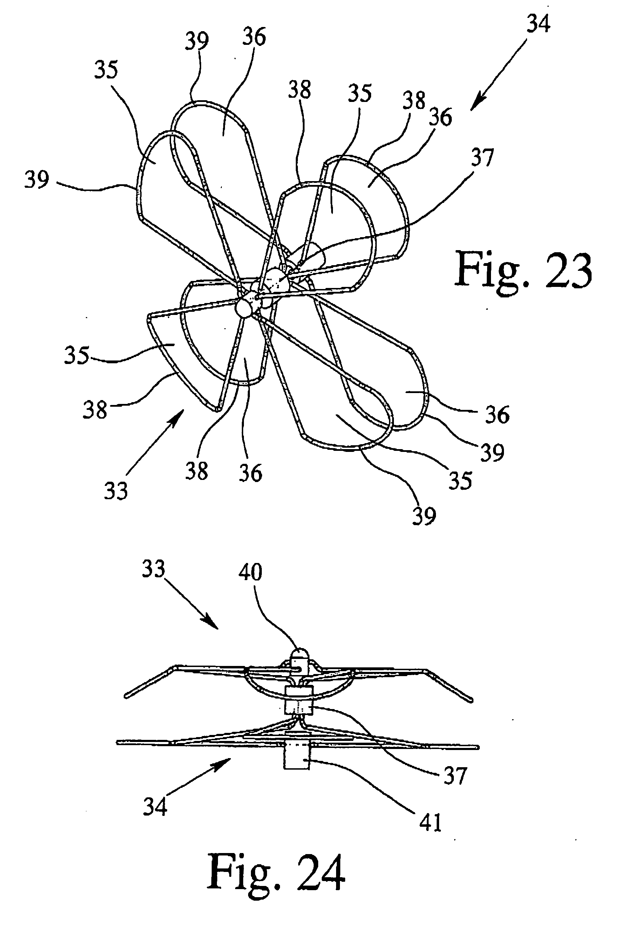

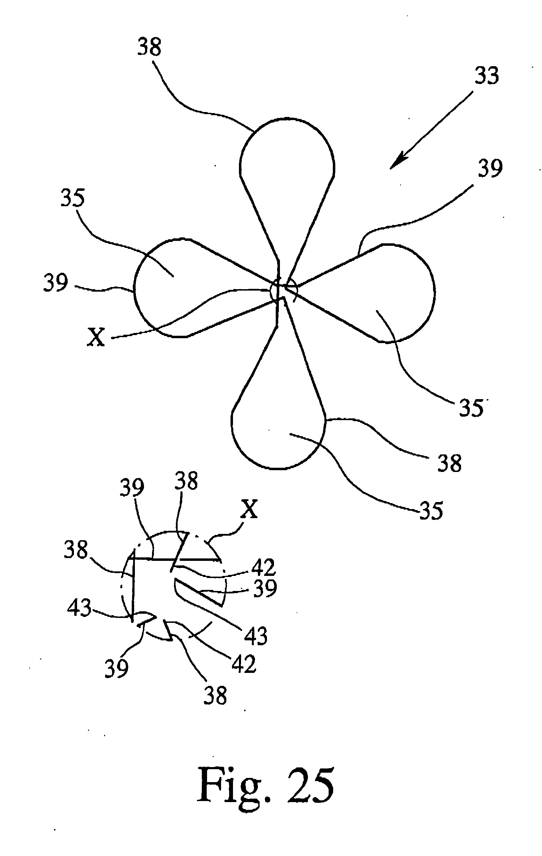

Anhand der Fig. 23 bis 27 wird eine weitere Ausführungsform eines Okkluders 7 beschrieben, der einen oberen schirmartigen Verschlußkörper 33 und einen unteren schirmartigen Verschlußkörper 34 aufweist. Die Verschlußkörper 33, 34 werden durch jeweils vier Segmente 35, 36 gebildet. Die Verschlußkörper 33, 34 sind durch ein Zwischenstück 37 miteinander verbunden. Die Segmente 35, 36 der Verschlußkörper 33, 34 werden jeweils durch zwei Drahtstücke 38, 39 gebildet, was in Fig. 25 exemplarisch für den oberen Verschlußkörper 33 dargestellt ist. Ein Drahtstück 38 bzw. 39 bildet dabei jeweils zwei gegenüberliegende Segmente 35 aus. Ebenso werden die Segmente 36 des unteren Verschlußkörpers 34 durch zwei Drahtstücke 38, 39 gebildet.

Zur Verbindung der Drahtstücke 38, 39 des oberen Verschlußkörpers 33 ist ein oberes Verbindungsstück 40 und zur Verbindung der Drahtstücke 38, 39 des unteren Verschlußkörpers 34 ist ein unteres Verbindungsstück 41 vorgesehen. Die Verbindungsstücke 40, 41 und das Zwischenstück 37 können als Kapazität ausgelegt werden. Darüber hinaus kann die erforderliche Kapazität in Form eines zusätzlichen Kondensatorbauteils zur Verfügung gestellt werden, das zwischen benachbarten Drahtenden 42, 43 der Drahtstücke 38, 39 des oberen Verschlußkörpers 33 und des unteren Verschlußkörpers 34 angebracht wird, was jedoch nicht im einzelnen dargestellt ist. Darüber hinaus ist es möglich, die Kapazität dadurch auszubilden, daß vorzugsweise die benachbarten Drahtenden 42, 43 der Drahtstücke 38, 39 eines Verschlußkörpers 33, 34 mit einem definiertem Abstand zueinander angeordnet sind. Dies ist exemplarisch für den oberen Verschlußkörper 33 in Fig. 25 anhand der Einzelheit X dargestellt. Hier ist es möglich, daß die Drahtenden 42, 43 parallel zueinander angeordnet sind.

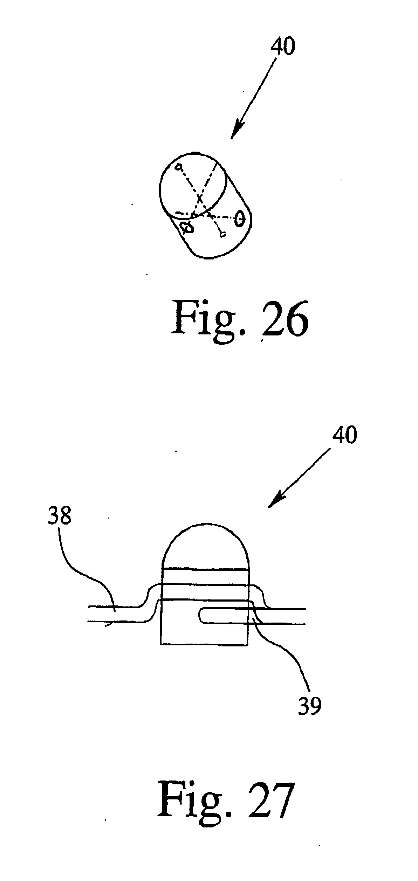

Das Verbindungsstück 40 für die Drahtstücke 38, 39 des oberen Verschlußkörpers 33 ist in den Fig. 26 und 27 dargestellt. In Fig. 27 ist der Verlauf der Drahtstücke 38, 39 durch das Verbindungsstück 40 hindurch dargestellt. Die Drahtstücke 38, 39 sind dabei durch Führung in dem Verbindungsstück 40 fixiert. Im übrigen sind die Drahtenden 42, 43 der Drahtstücke 38, 39 in dem Zwischenstück 37 fixiert, was sich aus Fig. 24 ergibt.

## Patentansprüche

1. Okkluder (7) zum Verschluß von Körperöffnungen (6, 6a) des menschlichen oder tierischen Körpers, insbesondere Okkluder (7) zum perkutanen transkatheter Verschluß von Vorhof-Septum-Defekten des menschlichen oder tierischen Herzens (1), mit wenigstens zwei im Verschlußzustand der Körperöffnung (6, 6a) auf gegenüberliegenden Seiten der Körperöffnung (6, 6a) angeordneten zumindest bereichsweise gegenüberliegenden Verschlußkörpern (8, 9) und mit wenigstens einem die Verschlußkörper (8, 9) miteinander verbindenden Zwischenstück (10), wobei das Zwischenstück (10) im Verschlußzustand zumindest bereichsweise durch die Körperöffnung (6, 6a) hindurchgeführt ist, wobei eine einseitige Verbindung zwischen dem Zwischenstück (10) und jedem Verschlußkörper (8, 9) vorgesehen ist, wobei das Zwischenstück (10) im Verschlußzustand mit jedem Verschlußkörper (8, 9) exzentrisch im Randbereich des Verschlußkörpers (8, 9) verbunden ist und wobei die im Verschlußzustand auf gegenüberliegenden Seiten der Körperöffnung (6, 6a) angeordneten gegenüberliegenden Verschlußkörper (8, 9) auf entgegengesetzten Seiten (A, B) mit dem Zwischenstück (10) verbunden sind, **dadurch gekennzeichnet, dass** der Okkluder durch einen an seinen Enden zusammenlaufenden einstückigen Draht (17) gebildet ist, wobei der Draht (17) auf den entgegengesetzten Seiten (A, B) jeweils zu einem einen außenliegenden Verschlußkörper (8, 9) bildenden Drahtring verformt ist, wobei der Drahtring zwei aufeinander zulaufende Drahtabschnitte (18, 19) aufweist, wobei die Drahtabschnitte (18, 19) radial in Richtung zum Mittelpunkt des Drahtrings abgebogen sind und in nebeneinander verlaufende Schenkel (20, 21) übergehen und wobei die Schenkel (20, 21) der beiden Verschlußkörper (8, 9) das innenliegende Zwischenstück (10) bilden.

2. Okkluder nach Anspruch 1, **dadurch gekennzeichnet, daß** das Zwischenstück (10) eine Abstufung (11) mit wenigstens zwei entgegengesetzt verlaufenden Schenkeln aufweist, wobei jeder Schenkel mit seinem freien Ende mit einem Verschlußkörper (8, 9) verbunden ist.

3. Okkluder nach Anspruch 1 oder 2, **dadurch gekennzeichnet, daß** die Schenkel des Zwischenstücks (10) dieselbe Länge aufweisen, so daß die Abstufung (11) im Verschlußzustand im wesentlichen zentrisch zu den auf gegenüberliegenden Seiten der Körperöffnung (6) angeordneten Verschlußkörpern angeordnet ist.

4. Okkluder nach einem der vorhergehenden Ansprüche, **dadurch gekennzeichnet, daß** die im Verschlußzustand auf gegenüberliegenden Seiten der Körperöffnung (6) angeordneten Verschlußkörper (8, 9) im wesentlichen einander überdecken.

5. Okkluder nach einem der vorhergehenden Ansprüche, **dadurch gekennzeichnet, daß** die Verschlußkörper (8, 9) und das Zwischenstück (10) aus einem einstückigen Draht (17) aus einer Formgedächtnis-Legierung gebildet oder aus einem Rohr aus einer Formgedächtnis-Legierung geschnitten sind.

6. Okkluder nach einem der vorhergehenden Ansprüche, **dadurch gekennzeichnet, daß** die Drahtabschnitte (18, 19) in parallel zueinander verlaufende Schenkel (20,21) übergehen.

7. Okkluder nach einem der vorhergehenden Ansprüche, **dadurch gekennzeichnet, daß** die Schenkel (20,21) der beiden Verschlußkörper (8, 9) im Bereich des Zwischenstücks (10), insbesondere im Bereich der Abstufung (11), miteinander verbunden sind.

8. Okkluder nach einem der vorhergehenden Ansprüche, **dadurch gekennzeichnet, daß** die Enden (23, 24) des Drahtes (17) einen Kondensator des Resonanzschwingkreises ausbilden.

9. Okkluder nach einem der vorhergehenden Ansprüche, **dadurch gekennzeichnet, daß** der Verschlußkörper (8, 9) eine Verschlußfläche (12, 13) aufspannt und daß die das Zwischenstück (10) bildenden Schenkel außerhalb der Verschlußfläche (12, 13) verlaufend angeordnet sind.

10. Okkluder nach einem der vorhergehenden Ansprüche, **dadurch gekennzeichnet, daß** wenigstens ein Verschlußkörper (8, 9) und/oder das Zwischenstück (10) und/oder der Okkluder (7) mit einem Gewebe umhüllt sind.

11. Okkluder nach einem der vorhergehenden Ansprüche, **dadurch gekennzeichnet, daß** das Zwischenstück (10) im Bereich der Abstufung (11) ummantelt ist und daß, vorzugsweise, die Ummantelung eine Führungsöffnung für einen Führungsdraht aufweist.

12. Okkluder nach einem der vorhergehenden Ansprüche, **dadurch gekennzeichnet, daß** der Okkluder wenigstens einen haken- oder ösenförmigen Angriffsabschnitt (14) für ein Implantationswerkzeug aufweist.

13. Okkluder nach einem der vorhergehenden Ansprüche, **dadurch gekennzeichnet, daß** der Okkluder (7) oder Teile des Okkluders (7) einen elektrischen Resonanzschwingkreis ausbilden, wobei wenigstens eine die Induktivität des Resonanzschwingkreises ausbildende Leiterschleife vorgesehen ist und der Okkluder (7) oder Teile des Okkluders (7) von der Leiterschleife ausgebildet werden.

14. Okkluder nach Anspruch 13, **dadurch gekennzeichnet, daß** wenigstens ein Verschlußkörper (8, 9) von der Leiterschleife gebildet wird.

15. Okkluder nach einem der Ansprüche 13 bis 14, **dadurch gekennzeichnet, daß** die Leiterschleife eine Kapazität des Resonanzschwingkreises ausbildet oder daß wenigstens ein separates eine Kapazität bildendes Bauteil vorgesehen ist, wobei das Bauteil mit der Leiterschleife kontaktiert ist.

## Claims

1. Occluder (7) for closing body openings (6, 6a) of the human or animal body, more particularly occluder (7) for the percutaneous transcatheter closure of atrial septum defects of the human or animal heart (1), with at least two closing elements (8, 9) arranged at least partially opposite each other on opposite sides of the body opening (6, 6a) when the body opening (6, 6a) is in the closed state, and with at least one intermediate piece (10) connecting the closing elements (8, 9) with each other, wherein in the closed state the intermediate piece (10) at least partially penetrates the body opening (6, 6a), whereby a unilateral connection between the intermediate piece (10) and each closing element (8, 9) is provided, whereby in the closed state the intermediate piece (10) is eccentrically connected to each closing element (8, 9) in the marginal area of the closing element (8, 9) and whereby the closing elements (8, 9) arranged opposite each other on opposite sides of the body opening (6, 6a) in the closed state are connected to the intermediate piece (10) on opposite sides (A, B), **characterised in that** the occluder is formed by a single wire (17) merging at its ends, whereby the wire (17) on opposite sides (A, B) is deformed into a wire ring forming an outer closing body (8, 9), whereby the wire ring has two converging wire sections (18, 19), whereby the wire sections (18, 19) are bent radially in the direction of the midpoint of the wire ring and become adjacently running arms (20, 21) and whereby the arms (20, 21) of the two closing elements (8, 9) form the inner intermediate piece (10).

2. Occluder according to claim 1, **characterised in that** the intermediate piece (10) has a stepped section (11) with at least two oppositely extending arms where each arm is connected with its free end to a closing element (8, 9).

3. Occluder according to claim 1 or 2, **characterised in that** the arms of the intermediate piece (10) are of the same length so that in the closed state the stepped section (11) is arranged essentially centrically to the closing elements arranged on opposite sides of the body opening (6).

4. Occluder according any one of the preceding claims, **characterised in that** in the closed position the closing elements (8, 9) which are arranged on opposite sides of the body opening (6) essentially overlap each other.

5. Occluder according to any one of the preceding claims, **characterised in that** the closing elements (8, 9) and the intermediate piece (10) is cut from of a single wire (17) made of a shape-memory alloy or from a tube made of a shape-memory alloy.

6. Occluder according to any one of the preceding claims, **characterised in that** the wire sections (18, 19) become arms (20, 21) which run in parallel to each other.

7. Occluder according to any one of the preceding claims, **characterised in that** the arms (20, 21) of the two closing elements (8, 9) are connected to each other in the area of the intermediate piece (10), more particularly in the area of the stepped section (11).

8. Occluder according to any one of the preceding claims, **characterised in that** the ends (23, 24) of the wire (17) form a capacity of the resonance oscillation circuit.

9. Occluder according to any one of the preceding claims, **characterised in that** the closing elements (8, 9) tension a closing area (12, 13) and **in that** the arms forming the intermediate piece (10) are arranged outside the closing area (12, 13).

10. Occluder according to any one of the preceding claims, **characterised in that** at least one closing element (8, 9) and/or the intermediate piece (10) and/or the occluder (7) are encased in a woven material.

11. Occluder according to any one of the preceding claims, **characterised in that** the intermediate piece (10) is encased in the area of the stepped section (11) and **in that**, preferably, the encasing has a guide opening for a guide wire.

12. Occluder according to any one of the preceding claims, **characterised in that** the occluder has at least one hook or eye-shaped grip section (14) for an implantation tool.

13. Occluder according to any one of the preceding claims, **characterised in that** the occluder (7) or parts of the occluder (7) form an electrical oscillation circuit, whereby at last one conductor loop forming the inductivity of the resonance oscillation circuit is provided and the occluder (7) or parts of the occluder (7) are formed by the conductor loop.

14. Occluder according to claim 13, **characterised in that** the conductor loop forms at least one closing element (8, 9).

15. Occluder according to claims 13 to 14, **characterised in that** the conductor loop forms a capacity of the resonance oscillation circuit or **in that** at least one separate capacity-forming component is provided, whereby the component is in contact with the conductor loop.

## Revendications

1. Obturateur (7) pour la fermeture d'orifices corporels (6, 6a) du corps humain ou animal, en particulier obturateur (7) pour la fermeture trans-cathéter percutanée de défauts du septum interauriculaire du coeur humain ou animal (1), comprenant au moins deux corps obturateurs (8, 9) au moins opposés par endroits et disposés, à l'état de fermeture de l'orifice corporel (6, 6a), sur des faces opposées de l'orifice corporel (6, 6a) et au moins une pièce intermédiaire (10) reliant les corps obturateurs (8, 9) entre eux, la pièce intermédiaire (10) passant, à l'état de fermeture, au moins par endroits dans l'orifice corporel (6, 6a), une liaison unilatérale étant prévue entre la pièce intermédiaire (10) et chaque corps obturateur (8, 9), la pièce intermédiaire (10) étant, à l'état de fermeture, reliée de manière excentrée à chaque corps obturateur (8, 9) sur la périphérie du corps obturateur (8, 9) et les corps obturateurs (8, 9) disposés à l'état de fermeture sur des faces opposées de l'orifice corporel (6, 6a) étant reliés sur des faces opposées (A, B) à la pièce intermédiaire (10), **caractérisé en ce que** l'obturateur est constitué par un fil (17) monobloc convergeant à ses extrémités, le fil (17) étant respectivement déformé sur les faces opposées (A, B) en un anneau de fil formant un corps obturateur extérieur (8, 9), l'anneau de fil présentant deux sections de fil (18, 19) se rejoignant, les sections de fil (18, 19) étant courbées radialement en direction du point central de l'anneau de fil et transitant dans des branches à extension adjacente (20, 21) et les branches (20, 21) des deux corps obturateurs (8, 9) formant la pièce intermédiaire interne (10).

2. Obturateur selon la revendication 1, **caractérisé en ce que** la pièce intermédiaire (10) présente un échelon (11) avec au moins deux branches s'étendant en sens inverse, chaque branche étant reliée par son extrémité libre à un corps obturateur (8, 9).

3. Obturateur selon la revendication 1 ou 2, **caractérisé en ce que** les branches de la pièce intermédiaire (10) présentent la même longueur, de sorte que l'échelon (11), à l'état de fermeture, est disposé sensiblement centré par rapport aux corps obturateurs disposés sur les faces opposées de l'orifice corporel (6).

4. Obturateur selon une des revendications précédents, **caractérisé en ce que** les corps obturateurs (8, 9) disposés, à l'état de fermeture, sur des faces opposées de l'orifice corporel (6) se recouvrent sensiblement.

5. Obturateur selon une des revendications précédents, **caractérisé en ce que** les corps obturateurs (8, 9) et la pièce intermédiaire (10) sont formés d'un fil monobloc (17) en alliage à mémoire de forme ou découpés dans un tube en alliage à mémoire de forme.

6. Obturateur selon une des revendications précédents, **caractérisé en ce que** les sections de fil (18, 19) transitent dans des branches (20, 21) s'étendant parallèlement les unes aux autres.

7. Obturateur selon une des revendications précédents, **caractérisé en ce que** les branches (20, 21) des deux corps obturateurs (8, 9) sont reliées entre elles au niveau de la pièce intermédiaire (10), en particulier au niveau de l'échelon (11).

8. Obturateur selon une des revendications précédents, **caractérisé en ce que** les extrémités (23, 24) du fil (17) constituent un condensateur du circuit oscillateur de résonance.

9. Obturateur selon une des revendications précédents, **caractérisé en ce que** le corps obturateur (8, 9) sous-tend une surface obturatrice (12, 13) et que les branches formant la pièce intermédiaire (10) sont disposées de manière à s'étendre en dehors de la surface obturatrice (12, 13).

10. Obturateur selon une des revendications précédents, **caractérisé en ce qu'**au moins un corps obturateur (8, 9) et/ou la pièce intermédiaire (10) et/ou l'obturateur (7) sont enrobés d'un textile.

11. Obturateur selon une des revendications précédents, **caractérisé en ce que** la pièce intermédiaire (10) est revêtue au niveau de l'échelon (11) et que le revêtement présente de préférence un orifice de passage pour un fil de guidage.

12. Obturateur selon une des revendications précédentes, **caractérisé en ce que** l'obturateur présente au moins une section de préhension (14) en forme de crochet ou d'oeillet pour un outil d'implantation.

13. Obturateur selon une des revendications précédentes, **caractérisé en ce que** l'obturateur (7) ou des éléments de l'obturateur (7) forment un circuit oscillant de résonance, au moins une boucle conductrice établissant l'inductivité du circuit oscillant de résonance et l'obturateur (7) ou des éléments de l'obturateur (7) étant formés par la boucle conductrice.

14. Obturateur selon la revendication 13, **caractérisé en ce qu'**au moins un corps obturateur (8, 9) est formé par la boucle conductrice.

15. Obturateur selon une des revendications 13 à 14, **caractérisé en ce que** la boucle conductrice établit une capacité du circuit oscillant de résonance ou qu'au moins une pièce séparée établissant une capacité est prévue, la pièce étant en contact avec la boucle conductrice.
